# EUROPEAN PATENT APPLICATION

(11) **EP 2 606 836 A1**
(43) Date of publication of application: **26.06.2013**
(21) Application number: 11010156.5
(22) Date of filing: 23.12.2011
(51) Int. Cl.: A61B 17/17

(54) **Targeting guide and surgical system comprising the targeting guide**

(71) Applicant: Zimmer GmbH, 8404 Winterthur (CH)
(72) Inventor: Senger, Reto, 8409 Winterthur (CH); Schwammberger, Andy, 4431 Bennwil (CH)
(74) Representative: Manitz, Finsterwald & Partner GbR

(57) **Abstract**

The present disclosure relates to a targeting guide to be used in the internal fixation for fractured long bones, comprising an arm (11) which is adapted and configured to be connected to an implant (13) which is adapted and configured to be inserted into a fractured long bone (15), a first guide member (17) and a second guide member (15) being arranged one inside the other and being displaceable with respect to each other in a displacement direction between a release configuration and a locking configuration, one of the guide members (17) being connected to the arm (11) such as to be immobile with respect to the arm (11), a first guide passage (19, 19') formed in the first guide member (17) and a second guide passage (27, 27') formed in the second guide member (25), the guide passages (19, 19', 27, 27') being adapted and configured to receive a tool (16) and/or a tool guide (23), wherein in the release configuration the guide passages (19, 27 and 19', 27', respectively) are aligned and define an alignment axis along which the tool (16) or tool guide (23) is freely movable with respect to the guide members (17, 25), and wherein in the locking configuration the guide passages (19, 27 and 19', 27', respectively) are misaligned and/or offset such as to clamp the tool (16) or tool guide (23) received in the guide passages (19, 19', 27, 27'), and an actuation mechanism adapted and configured to displace the guide members (17, 25) with respect to each other between the release configuration and the locking configuration.

## Description

The present disclosure relates to a targeting guide to be used in the internal fixation for fractured long bones.

Such targeting guides facilitate a correct insertion and alignment of an implant or implant system that serves to stabilize the bone. In particular, said guides foster an assembly of implant systems which are composed of more than one component. Moreover, targeting guides are also used in many cases during preparation of the bone prior to insertion of the implant or implant system or components thereof.

Here, a novel targeting guide is suggested that supports a reliable and easy preparation of the bone and/or insertion, alignment and/or assembly of an implant or implant system.

The targeting guide according to claim 1 is provided with an arm which is adapted and configured to be connected to an implant which is adapted and configured to be inserted into a fractured long bone. Further, the targeting guide comprises a first guide member and a second guide member being arranged one inside the other and being displaceable with respect to each other - e.g. by a rotation or a linear relative movement of the first and/or second guide members - in a displacement direction between a release configuration and a locking configuration. One of the guide members is connected to the arm such as to be immobile with respect to the arm. A first guide passage is formed in the first guide member and a second guide passage is formed in the second guide member, the guide passages being adapted and configured to receive a tool and/or a tool guide, wherein in the release configuration the guide passages are aligned and define an alignment axis along which the tool or tool guide is freely movable with respect to the guide members, and wherein in the locking configuration the guide passages are misaligned and/or offset such as to clamp the tool or tool guide received in the guide passages. The targeting guide further comprises an actuation mechanism adapted and configured to displace the guide members with respect to each other between the release configuration and the locking configuration.

Clamping of the tool or the tool guide to the first and second guide members allows - inter alia - temporarily interrupting the process of preparing the bone and/or inserting, aligning or assembling the implant or the implant system if necessary without compromising the success of the surgery, since e.g. specifically selected geometrical settings of the components involved are reliably preserved.

According to an embodiment of the targeting guide, the guide passages are coaxially aligned with each other in the release configuration of the guide members, so as to allow the passage of the tool or the tool guide without hindrance.

In another embodiment of the targeting guide, the guide passages are - at least partially - overlapping each other when viewed along the alignment axis in the locking configuration of the guide members. In other words, the guide passages do not fall exactly in line in this configuration but their contours intersect to achieve the desired clamping effect.

In yet another embodiment of the targeting guide, the guide members are movable with respect to each other exclusively in the displacement direction, in particular along a linear displacement axis. In other words, the degree of freedom as regards a relative movement of the guide members is limited to the displacement direction in order to minimize the risk of inadvertent misuse of the targeting guide. In particular, the guide members are displaceable with respect to each other along a linear displacement axis. However, it is also feasible to allow a relative rotational displacement.

According to another embodiment, the guide member which is connected to the arm is formed integrally with the arm such that the guide member and the arm are made in one piece.

According to a further embodiment, the first guide member is connected to the arm and the second guide member is received within the first guide member.

Exemplarily, the first guide member is a housing arranged over the second guide member, and/or the second guide member is a barrel.

In yet another embodiment, the guide passages are a first pair of guide passages and the alignment axis defined by the first pair of guide passages in the release configuration is a first alignment axis, and wherein the guide members have at least one second pair of guide passages which in the release configuration define a second alignment axis, the second alignment axis being non-parallel to the first alignment axis. Providing the first and second alignment axis allows using the targeting guide with different implants that are optimized for different anatomical situations.

According to a simple yet effective embodiment, the guide passages are guide bores.

According to another embodiment of the targeting guide, the actuation mechanism comprises an actuation member connected to one guide member and a transfer mechanism coupling the actuation member to the other guide member to selectively control the displacement of the guide members. Exemplarily, the actuation member is fixedly connected to the guide member with respect to the displacement direction. The transfer mechanism may be adapted and configured to transfer an actuation movement of the actuation member into a displacement of the guide members with respect to each other in the displacement direction between the release configuration and the locking configuration.

According to a further embodiment, the actuation movement of the actuation member is a rotational actuation movement. In particular, the transfer mechanism is adapted and configured to transfer a rotational actuation movement of the actuation member into a linear displacement of the guide members with respect to each other along a linear displacement axis.

By way of example, the relative displacement of the guide members is achieved if the transfer mechanism couples the actuation member to the guide member which is connected to the arm. The transfer mechanism comprises in particular a restraint for coupling the actuation member to the other guide member. A restraint in that sense comprises e.g. a ramp-mechanism, a cam/cam-follower-combination, a threaded coupling or a male/female arrangement. It is also conceivable that the restraint comprises at least one pin-slot-arrangement having a pin and a slot, the pin being guided in the slot and the slot extending, in particular by less than 180°, in particular by less than 90°, as a helical segment around a linear displacement axis, such that moving the pin along the slot causes the pin to move or to be displaced along the displacement axis.

In an embodiment, the pin-slot-arrangement comprises two symmetrically arranged slots for the pin, each of the two slots extending, in particular by less than 180°, in particular by less than 90°, as a helical segment around the linear displacement axis, the two helical segments starting from a common point in the same direction as viewed along the linear displacement axis. Thus, in this embodiment the direction of displacement effected by rotating the actuation member is independent on the rotational direction of the actuation member, thereby making the use of the actuation mechanism easier. In other words, the actuation member can be rotated either clockwise or anti-clockwise, thereby in both cases effecting a displacement in the same linear direction.

In particular, the pin is connected to the actuation member and the slot is formed in the guide member, or vice versa.

According to another embodiment of the targeting guide, the guide member to which the actuation member is coupled by the transfer mechanism has an extension which extends into the actuation member and which is adapted and configured to receive the other guide member during assembly. In particular, the extension is provided with at least one slot for guiding a pin connected to the actuation member, the pin and the slot being parts of the transfer mechanism. In an embodiment, the extension comprises a hollow cylinder portion connected to an end of the guide member facing the actuation member, the hollow cylinder portion comprising a wall which is provided with a coupling section for coupling the actuation member to the guide member.

To avoid inadvertent disassembly, securing means are provided in another embodiment of the targeting guide, the securing means being adapted and configured to prevent the actuation member from being unintentionally detached from the guide member to which the actuation member is coupled by the transfer mechanism. In particular, the securing means are part of the transfer mechanism. It is also conceivable that the restraint of the transfer mechanism is adapted and configured to form the securing means.

According to a further embodiment, the securing means comprise a thread portion formed at the guide member to which the actuation member is coupled by the transfer mechanism, and a thread portion formed at the actuation member, the thread portions being adapted and configured to cooperate during a first phase of assembly in which the actuation member and the guide member are screwed together before being coupled, and to be disengaged in a second phase of assembly in which the actuation member is coupled to the guide member by the transfer mechanism. Particularly, the actuation member and the guide member to which the actuation member is coupled by the transfer mechanism each have a coupling portion adjacent the thread portion, the coupling portions forming the transfer mechanism and being arranged with respect to the thread portions such that in the first phase of assembly the coupling portions lie on opposite sides of the cooperating thread portions. It is e.g. possible to form the thread portion and the coupling portion of the guide member at a hollow cylinder portion of an extension of the guide member, the hollow cylinder portion having a longitudinal axis and the thread portion and the coupling portion being arranged one after another along the longitudinal axis.

Further, a novel targeting system is suggested that supports a reliable and easy insertion, alignment and/or assembly of an implant or implant system in fractured long bones or their preparation for the aforementioned procedures.

Said targeting system to be used in the internal fixation for fractured long bones comprises a targeting guide according to any of the embodiments described above, and at least one tool and/or tool guide to be received in the guide passages of the targeting guide. In particular, the tool is a drill or a bone screw and/or the tool guide is a sleeve or a cannula.

The present disclosure also relates to a surgical system to be used in the internal fixation for fractured long bones, comprising at least one implant which is adapted and configured to be inserted into a fractured long bone, wherein the implant has at least one implant passage, a targeting guide according to any of the embodiments described above, wherein the arm of the targeting guide is adapted and configured to be connected to the implant when the implant is inserted into the bone, and wherein at least one alignment axis of the targeting guide is aligned with the implant passage when the arm of the targeting guide is connected to the implant. In particular, the implant has a longitudinal axis, the implant passage of the implant extending under an angle or oblique with respect to the longitudinal axis of the implant, i.e. non-parallel to the longitudinal axis of the implant.

According to an embodiment of the surgical system, the system comprises at least two implants, each implant having an implant axis and an implant passage which has an orientation with respect to the implant axis, the orientation of the implant passages of the two implants being different from each other, wherein the targeting guide has at least two pairs of guide passages, the respective alignment axis of the two pairs being non-parallel, and wherein for each implant the implant passage is aligned with at least one alignment axis of the targeting guide when the arm of the targeting guide is connected to that implant. In particular, the implant is a nail, more specifically a medullary nail.

The different embodiments of the targeting guide, the targeting system and the surgical system described above in accordance with the scope of the independent claim and the features realized there can naturally be combined with one another.

Further embodiments of the disclosure are also recited in the dependent claims, the description and the drawings.

Further areas of applicability of the present disclosure will become apparent from the detailed description provided hereinafter. It should be understood that the detailed description and specific examples are intended for the purpose of illustration only and are not intended to limit the scope of the disclosure in any way. The figures are simplified and schematic. Details not necessary for the understanding of the invention are omitted.

The present disclosure will be explained in more detail and become fully understood from the detailed description and the accompanying drawings, wherein
- Fig. 1: depicts an embodiment of the targeting guide in accordance with the present disclosure coupled to an implant disposed in a bone during the process of inserting a screw in the bone,
- Fig. 2: shows another embodiment of the targeting guide, and
- Fig. 3: shows yet another embodiment of the targeting guide.

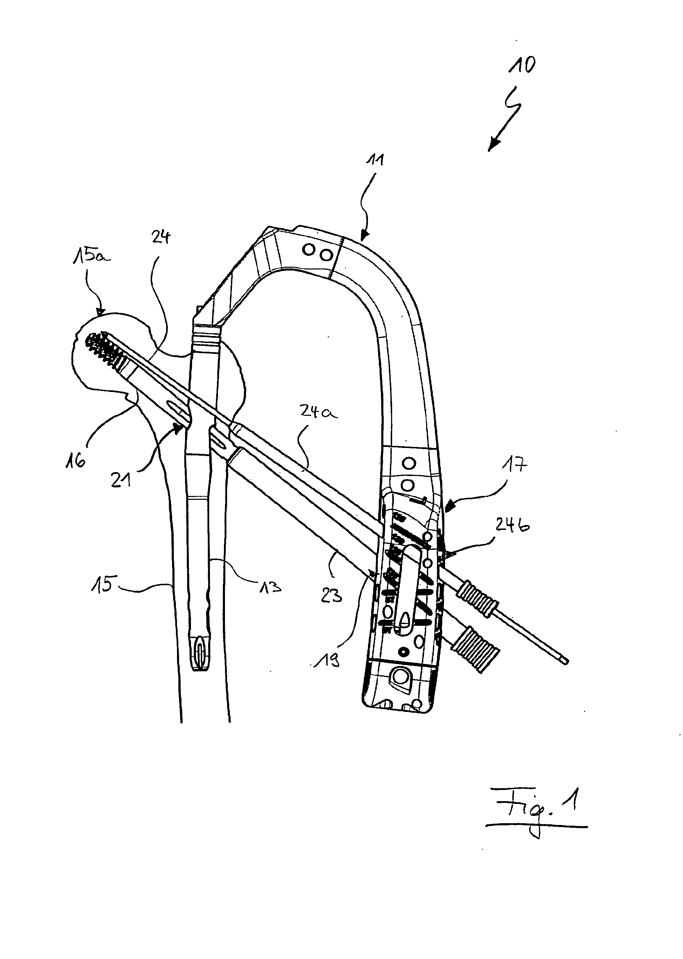

Fig. 1 shows a targeting guide 10 comprising an arm 11 and a first guide member 17. Arm 11 is coupled to an implant 13 - in the present case a medullary nail - which is disposed in the proximal end of a femur 15. In the present example, the patient requires a fixation of a head 15a of femur 15 to the rest of femur 15. It should be understood, that targeting guide 10 and variants thereof can be used to stabilize other bones, in particular long bones.

To this end, nail 13 has already been fixed in the medullary canal of femur 15. A screw 16 has been inserted in a passage 21 of nail 13 and screwed in head 15a using a sleeve 23. To ensure that an anatomical correct fixation of head 15a to femur 15 can be carried out, it is important that all the components involved are properly aligned and positioned. In particular, the position and orientation of passage 21 of nail 13 has to be properly aligned with respect to femur 15 to allow the anatomical correct fixation of head 15a to femur 15 using screw 16. Orienting passage 21 can be carried out prior to coupling arm 11 to nail 13. However, it is also possible to change the position of nail 13 and/or to orient passage 21 using targeting guide 10.

Once nail 13 is positioned and oriented correctly, screw 16 has to be inserted to provide a coupling between head 15a of femur 15 and nail 13. During the surgery, the view on passage 21 is in most cases obstructed, so that the insertion of screw 16 is rather complicated. Targeting guide 10 is provided to facilitate the process of inserting screw 16. Arm 11 of targeting guide 10 provides a fixed geometrical relationship between passage 21 and a first guide passage 19 of first guide member 17. Pushing screw 16 with sleeve 23 through first guide passage 19 leads automatically to a correct insertion of screw 16 in passage 21, as first guide passage 19 and passage 21 are aligned along an alignment axis, assuming that arm 11 is properly attached to nail 13. Proper attachment of arm 11 to nail 13 can be achieved by providing suitable coupling means (not shown).

Sleeve 23 allows not only to insert screw 16 in passage 21 but also facilitates screwing it into head 15a thereby establishing a fixed relationship between head 15a and bone 15 via nail 13. Fixation of screw 16 to nail 13 can be accomplished by conventional fixation means.

It should be understood that a tool can be inserted in passage 21 using targeting guide 10 in an analogous manner as described above in connection with screw 16. Such a tool can e.g. be a drill used during a preparatory step before inserting screw 16 connecting head 15a to nail 13.

In order to foster the stability of the orientation of targeting guide 10 relative to femur 15 and its head 15a during the insertion process outlined above, a stabilization pin 24 is provided. Said pin 24 is directed and stabilized by stabilization sleeve 24a which in turn is disposed in a stabilization pin passage 24b that has a known geometrical position and orientation with respect to first guide member 17. Once pin 24 has been inserted in femur 15 and/or its head 15a, pin 24 reliably inhibits relative rotations of the components described above.

Specific circumstances may require that the insertion process of screw 16 has to be halted temporarily. To make sure e.g. that screw 16 does not slip from passage 21 - which might happen especially if a threaded portion of screw 16 has not yet bitten into the bone material of head 15a - and/or to prevent unintentional misalignment of targeting guide 10, a mechanism is provided to fix screw 16 or sleeve 23 temporarily but reliably to first guide member 17.

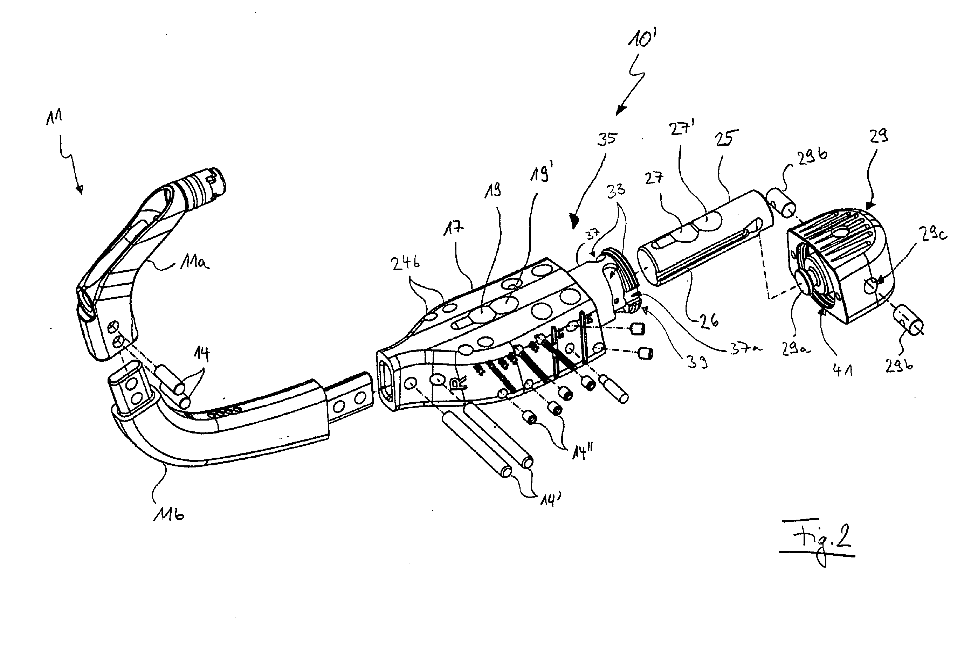

Fig. 2 shows an embodiment 10' of a targeting guide according to the present disclosure in a disassembled state. Its arm 11 comprises two arm segments 11a, 11b which can be coupled by pins 14. Arm segment 11b is - in an assembled state of targeting guide 10' - coupled via pins 14' to first guide member 17 which in turn is provided with a number of stabilization pin passages 24b with different orientations to allow the use of one or more stabilization pin/sleeve-combinations 24, 24a as needed. Pins 14" are provided for fixing stabilization pin/ sleeve-combinations 24, 24a relative to first guide member 17.

Further, first guide member 17 is provided with guide passages 19, 19' to receive screw 16 coupled to sleeve 23. Passages 19, 19' are oriented in such a way that they fall in line with passage 21 of a corresponding nail 13 - when arm 11 is attached correctly to nail 13 - thereby each defining an alignment axis along which screw 16 is advanced during the insertion process. However, passages 19, 19' do not define parallel alignment axes. Each passage 19, 19' defines - together with a correspondingly oriented passage 21 of a suitable nail 13 - a specific alignment axis that allows taking patient specific anatomical situations into account. In other words, first guide member 17 can be used in combination with at least two different nails 13. It should be understood that first guide member 17 can be provided with an arbitrary number of guide passages 19, 19'.

First guide member 17 is provided with a channel in its interior that receives a second guide member 25 of essentially cylindrical shape. A portion of the channel of first guide member 17 is partly defined by a cylindrical wall 37 which forms a cylindrical extension 35 of first guide member 17 facing the actuation member 29. In an assembled state of targeting guide 10', second guide member 25 is disposed slidably in said channel. Grooves 26 on second guide member 25 cooperating with suitable protrusions in the channel prevent a rotation of second guide member 25 when positioned within the channel of first guide member 17. Second guide member 25 and first guide member 17 can assume at least two different configurations as regards their relative position: (1) a release configuration and (2) a locking configuration.

In the release configuration, guide passages 27, 27' provided on second guide member 25 are aligned with guide passages 19, 19', respectively, of first guide member 17. In other words, passages 27, 19 and 27', 19' are coaxially arranged in the release configuration. Screw 16 or sleeve 23 disposed in said passages 27, 19 or 27', 19' can therefore be moved without hindrance along the respective alignment axis.

In case the insertion process of screw 16 and/or sleeve 23 has to be interrupted temporarily, second guide member 25 is moved linearly with respect to first guide member 17 thereby also displacing passages 27, 19 and 27', 19', respectively, relative to each other. Figuratively speaking, passages 27, 19 and 27', 19' then overlap at least partly, since they are no longer coaxially aligned. At a given point, an inner surface of passage 27 or 27' starts to press screw 16 or sleeve 23 against an inner surface of passage 19 or 19', respectively, thereby creating clamping forces that reliably fix the position of screw 16 or sleeve 23 relative to first guide member 17 and in consequence also relative to nail 13, femur 15 and head 15a. Second guide member 25 and first guide member 17 have then reached the locking configuration.

To effect the displacement of the second guide member 25 within first guide member 17, targeting guide 10' is provided with an actuation mechanism comprising actuation member 29. Actuation member 29 is rotatably coupled via connecting pin 29a to second guide member 25. Although a free relative rotation between actuation member 29 and second guide member 25 is allowed, said components 29, 25 are coupled in essence fixedly as regards linear displacements.

In the course of the assembly of the targeting guide 10', second guide member 25 is inserted in the channel of first guide member 17. Actuation member 29 is coupled to guide member 25 and transfer pins 29b are disposed in corresponding holes 29c provided on actuation member 29. Then, actuation member 29 is placed over extension 35 which consequently extends into member 29 in an assembled state. Since actuation member 29 is provided with a threaded portion 41 that matches a complementary threaded portion 39 on wall 37 of extension 35, member 29 can be secured to member 17. The process of screwing member 29 onto extension 35 is continued until threaded portion 41 has been translated beyond threaded portion 39. In this situation, actuation member 29 can be rotated without being automatically translated axially. Axial movements of actuation member 29 are only limited in one direction by threaded portions 39, 41 and in the opposite direction by the surface of the first guide member 17 from which extension 35 protrudes.

A transfer mechanism based on restricted guidance is provided to obtain a defined axial movement of second guide member 25 coupled to actuation member 29 in response to a rotational actuation movement of actuation member 29. In order to assemble the transfer mechanism, securing pins 29b are aligned with grooves 37a cutting through threaded portion 39 provided on wall 37. Then, actuation member 29 is pushed in an axial direction until pins 29b reach slots 33 extending helically around the axial displacement axis of second guide member 25. Cooperation of pins 29b with slots 33 translates a rotation of actuation member 29 in a well defined movement of the second guide member 25 along the axial displacement axis.

It should be understood that alternative kinds of transfer mechanisms may be employed to translate an actuation of member 29 into a suitable displacement of second guide member 25 relative to first guide member 17 to cause the desired clamping forces. These clamping forces can in principle also be obtained by rotating second guide member 25 relative to first guide member 17. In general terms, the actuation movement and the displacement provoked thereby can both comprise rotational and/or translational components depending on the construction of the chosen transfer mechanism.

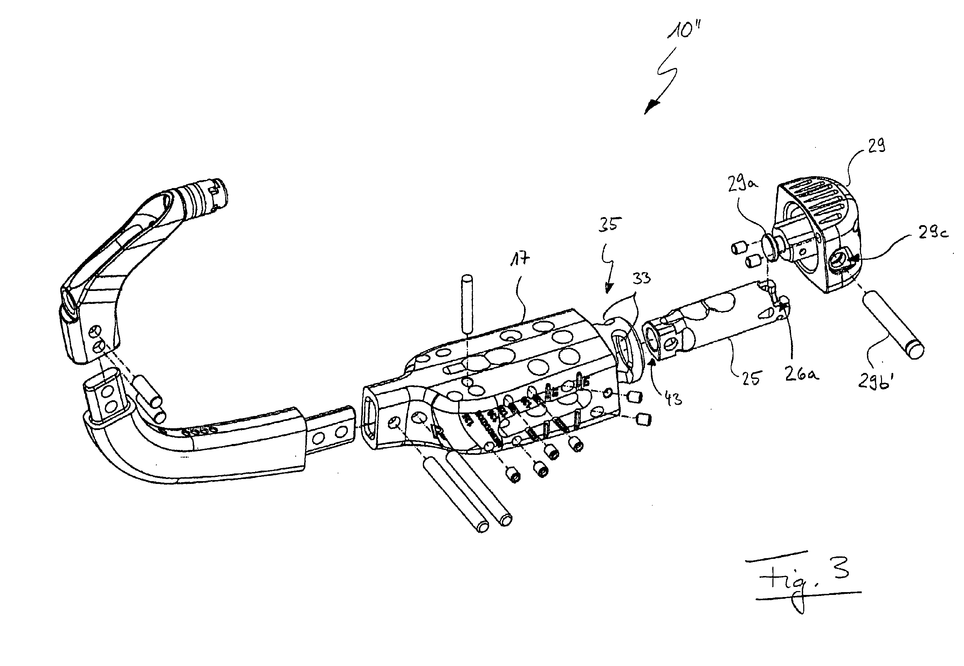

Fig. 3 shows another embodiment 10" of a targeting guide according to the present disclosure in a disassembled state. Many components of targeting guide 10" are similar to those of targeting guide 10'. Differences exist, however, in particular with respect to the actuation mechanism and the transfer mechanism.

The perspective of Fig. 3 provides an unobstructed view on details of the connection between second guide member 25 and actuation member 29 via connecting pin 29a placed in a notch 26a of member 25. The connection described above is essentially the same as that of targeting guide 10'.

Second guide member 25 of targeting guide 10" is not provided with grooves 26. As it can be inferred from the contour of an end face 43 of member 25 visible in Fig. 3, rotational displacements are suppressed by a non-rotational symmetry of - at least parts of - the body of second guide member 25 and a complementary cross-section of the channel within first guide member 17 which receives second guide member 25 in an assembled state of targeting guide 10".

Although the transfer mechanism of targeting guide 10" is also based on a restricted guidance provided by a pin-slot-cooperation, actuation member 29 is secured to first guide member 17 differently. When assembling targeting guide 10", at first second guide member 25 (already coupled to member 29) is disposed in the channel of first guide member 17. Then, actuation member 29 is placed over extension 35 of first guide member 17. To secure member 29 to member 17, transfer pin 29b' in inserted in holes 29c, thereby passing through slots 33 in wall 37. Transfer pin 29b' therefore secures actuation member 29 to first guide member 17 and at the same time represents a component of the mechanism that translates a rotational actuation movement of member 29 in a translational movement of second guide member 25.

All anatomical terms relating to directions and locations, such as anterior, posterior, medial, lateral, proximal, distal and sagittal, refer to an implanted state of the components and implants described above.

The description of the disclosure is merely exemplary in nature, and, thus, variations that do not depart from the gist of the disclosure are intended to be within the scope of the disclosure.

### List of reference numerals:

- 10, 10', 10": targeting guide
- 11: arm
- 11a, 11b: arm segment
- 13: nail
- 14, 14', 14": pin
- 15: femur
- 15a: head of the femur
- 16: screw
- 17: first guide member
- 19, 19': guide passage of first guide member
- 21: passage of implant
- 23: sleeve
- 25: second guide member
- 24: stabilization pin
- 24a: stabilization sleeve
- 24b: stabilization pin passage
- 26: groove
- 26a: notch
- 27, 27': guide passage of second guide member
- 29: actuation member
- 29a: connecting pin
- 29b, 29b': transfer pin
- 29c: hole
- 33: slot
- 35: extension
- 37: wall
- 37a: groove
- 39: thread portion of first guide member
- 41: thread portion of actuation member
- 43: end face of second guide member

## Claims

1. Targeting guide to be used in the internal fixation for fractured long bones, comprising
an arm (11) which is adapted and configured to be connected to an implant (13) which is adapted and configured to be inserted into a fractured long bone (15),
a first guide member (17) and a second guide member (25) being arranged one inside the other and being displaceable with respect to each other in a displacement direction between a release configuration and a locking configuration, one of the guide members (17) being connected to the arm (11) such as to be immobile with respect to the arm (11),
a first guide passage (19, 19') formed in the first guide member (17) and a second guide passage (27, 27') formed in the second guide member (25), the guide passages (19, 19', 27, 27') being adapted and configured to receive a tool (16) and/or a tool guide (23), wherein in the release configuration the guide passages (19, 27 and 19', 27', respectively) are aligned and define an alignment axis along which the tool (16) or tool guide (23) is freely movable with respect to the guide members (17, 25), and wherein in the locking configuration the guide passages (19, 27 and 19', 27', respectively) are misaligned and/or offset such as to clamp the tool (16) or tool guide (23) received in the guide passages (19, 19', 27, 27'), and
an actuation mechanism adapted and configured to displace the guide members (17, 25) with respect to each other between the release configuration and the locking configuration.

2. Targeting guide according to claim 1,
wherein in the release configuration of the guide members (17, 25), the guide passages (19, 27 and 19', 27', respectively) are coaxially aligned with each other, and/or
wherein in the locking configuration of the guide members (17, 25), the guide passages (19, 27 and 19', 27', respectively) are overlapping each other when viewed along the alignment axis.

3. Targeting guide according to claim 1 or 2,
wherein the guide members (17, 25) are movable with respect to each other exclusively in the displacement direction, in particular along a linear displacement axis, and/or
wherein the guide members (17, 25) are displaceable with respect to each other along a linear displacement axis.

4. Targeting guide according to any of claims 1 to 3,
wherein the first guide member (17) is a housing arranged over the second guide member (25), and/or wherein the second guide member (25) is a barrel.

5. Targeting guide according to any of claims 1 to 4,
wherein the guide passages are a first pair of guide passages (19, 27) and the alignment axis defined by the first pair of guide passages (19, 27) in the release configuration is a first alignment axis, and wherein the guide members (17, 25) have at least one second pair of guide passages (19', 27') which in the release configuration define a second alignment axis, the second alignment axis being non-parallel to the first alignment axis.

6. Targeting guide according to any of claims 1 to 5,
wherein the actuation mechanism comprises an actuation member (29) connected to one guide member (25) and a transfer mechanism (33, 29b, 29b') coupling the actuation member (29) to the other guide member (17).

7. Targeting guide according to claim 6,
wherein the transfer mechanism (33, 29b, 29b') is adapted and configured to transfer an actuation movement of the actuation member (29) into a displacement of the guide members (17, 25) with respect to each other in the displacement direction between the release configuration and the locking configuration.

8. Targeting guide according to claim 6 or 7,
wherein the actuation movement of the actuation member (29) is a rotational actuation movement.

9. Targeting guide according to claim 8,
wherein the transfer mechanism (33, 29b, 29b') is adapted and configured to transfer a rotational actuation movement of the actuation member (29) into a linear displacement of the guide members (17, 25) with respect to each other along a linear displacement axis.

10. Targeting guide according to any of claims 6 to 9,
wherein the transfer mechanism (33, 29b, 29b') comprises a restraint for coupling the actuation member (29) to the other guide member (17).

11. Targeting guide according to claim 10,
wherein the restraint comprises at least one pin-slot-arrangement having a pin (29b, 29b') and a slot (33), the pin (29b, 29b') being guided in the slot (33) and the slot (33) extending, in particular by less than 180°, in particular by less than 90°, as a helical segment around a linear displacement axis, such that moving the pin (29b, 29b') along the slot (33) causes the pin (29b, 29b') to be displaced along the displacement axis,
wherein in particular the pin-slot-arrangement has two symmetrically arranged slots (33) for the pin (29b, 29b'), each of the two slots (33) extending, in particular by less than 180°, in particular by less than 90°, as a helical segment around the linear displacement axis, the two helical segments starting from a common point in the same direction as viewed along the linear displacement axis.

12. Targeting system to be used in the internal fixation for fractured long bones, comprising
a targeting guide according to any of claims 1 to 11, and
at least one tool (16) and/or tool guide (23) to be received in the guide passages (19, 19', 27, 27') of the targeting guide.

13. Surgical system to be used in the internal fixation for fractured long bones, comprising
at least one implant (13) which is adapted and configured to be inserted into a fractured long bone (15),
wherein the implant (13) has at least one implant passage (21),
a targeting guide according to any of claims 1 to 27,
wherein the arm (11) of the targeting guide is adapted and configured to be connected to the implant (13) when the implant (13) is inserted into the bone (15), and
wherein at least one alignment axis of the targeting guide is aligned with the implant passage (21) when the arm (11) of the targeting guide is connected to the implant (13).

14. Surgical system according to claim 13,
wherein the system comprises at least two implants (13), each implant (13) having an implant axis and an implant passage (21) which has an orientation with respect to the implant axis, the orientation of the implant passages (21) of the two implants (13) being different from each other, wherein the targeting guide has at least two pairs of guide passages (19, 27 and 19', 27', respectively), the respective alignment axis of the two pairs (19, 27, 19', 27') being non-parallel, and wherein for each implant (13) the implant passage (21) is aligned with at least one alignment axis of the targeting guide when the arm (11) of the targeting guide is connected to that implant (13).

15. Surgical system according to claim 13 or 14,
wherein the implant is a nail, in particular a medullary nail (13).
